# EUROPÄISCHE PATENTSCHRIFT

(11) **EP 3 492 954 B1**
(45) Veröffentlichungstag und Bekanntmachung des Hinweises auf die Patenterteilung: **21.02.2024**
(21) Anmeldenummer: 18208520.9
(22) Anmeldetag: 27.11.2018
(51) Int. Cl.: G02B 27/14, G02B 23/24, G02B 5/20

(54) **OPTIKANORDNUNG FÜR EIN ENDOSKOP UND ENDOSKOP MIT EINER SOLCHEN OPTIKANORDNUNG**
LENS ASSEMBLY FOR AN ENDOSCOPE AND ENDOSCOPE WITH SUCH A LENS ASSEMBLY
DISPOSITIF OPTIQUE POUR UN ENDOSCOPE ET ENDOSCOPE DOTÉ D'UN TEL DISPOSITIF OPTIQUE

(30) Priorität: 27.11.2017 DE 102017127931
(43) Veröffentlichungstag der Anmeldung: 05.06.2019
(73) Patentinhaber: Henke-Sass, Wolf GmbH, 78532 Tuttlingen (DE)
(72) Erfinder: Rehe, Oliver, 78573 Wurmlingen (DE)
(74) Vertreter: Patentanwälte Geyer, Fehners & Partner mbB

(56) Entgegenhaltungen:
- EP-A2- 2 693 242
- US-A1- 2011 205 651
- US-A1- 2016 370 580

## Beschreibung

Die vorliegende Erfindung betrifft eine Optikanordnung für ein Endoskop und ein Endoskop mit einer solchen Optikanordnung.

Die US 2011/205651 A1 beschreibt eine Optikanordnung für ein Endoskop, wobei die Optikanordnung ein vor ihrem distalen Ende befindliches Objekt entlang eines eine optische Achse aufweisenden Hauptstrahlengangs zum proximalen Ende der Optikanordnung hin abbildet und wobei die Optikanordnung sowohl für Licht aus dem sichtbaren Spektrum als auch für Licht aus dem nahen Infrarot ausgelegt ist und im Hauptstrahlengang eine Aperturblende mit einer Schicht, die Licht aus dem nahen Infrarot transmittiert, aufweist. Die US 2016/370580 A1 beschreibt eine als Infrarotkantenfilter ausgebildete Schicht, die Infrarotstrahlung nicht transmittiert. Die EP 2 693 242 A2 beschreibt Infrarotfilter, die auf der Pixel-Ebene eines Detektors vorgesehen sind, wobei die Infrarotfilter als plane Filterelemente ausgebildet sind.

Wenn mittels der Optikanordnung für ein Endoskop sowohl eine Abbildung im sichtbaren Spektrum als auch im nahen Infrarot durchgeführt werden soll, ist es häufig erwünscht, für die Abbildung im sichtbaren Bereich eine hohe Schärfentiefe bereitzustellen. Im nahen Infrarotbereich liegt in der Regel ein schwaches Signal vor, da in diesem Wellenlängenbereich z.B. Fluoreszenzdiagnostik betrieben wird. Daher ist es für Licht aus dem nahen Infrarot häufig wünschenswert, dass eine größtmögliche Transmission durch die Optikanordnung bereitgestellt wird.

Diese Eigenschaften für Licht aus dem sichtbaren Wellenlängenbereich und Licht aus dem nahen Infrarot sollen möglichst so bereitgestellt werden, dass keine anderen optischen Eigenschaften der Abbildung im sichtbaren Wellenlängenbereich und der Abbildung im nahen Infrarot verschlechtert werden.

Die Erfindung ist im Anspruch 1 sowie im Anspruch 13 definiert. Vorteilhafte Weiterbildungen sind in den abhängigen Ansprüchen angegeben.

Die Reflexionsschicht ist gegenüber der optischen Achse geneigt. Sie weist also einen Winkel von ungleich 90° zur optischen Achse auf.

Erfindungsgemäß wird effektiv die Erzeugung von unerwünschten Geisterbildern verhindert, die auftreten, wenn das an der Aperturblende reflektierte Licht aus dem sichtbaren Spektrum in sich zurückreflektiert wird. Durch die geneigte Reflexionsschicht wird erfindungsgemäß das reflektierte Licht aus dem sichtbaren Spektrum nicht mehr in sich zurückreflektiert, sondern zur Seite hin reflektiert. Damit ist es möglich, an den entsprechenden seitlichen Stellen Strahlfallen auszubilden, so dass das reflektierte Licht nicht zu Geisterbildern führen kann.

Die Neigung der Reflexionsschicht ist so gewählt, dass Lichtstrahlen aus dem sichtbaren Spektrum, die parallel zur optischen Achse auf die Reflexionsschicht treffen, so reflektiert werden, dass sie nicht mehr parallel zur optischen Achse verlaufen. Insbesondere kreuzen die reflektierenden Lichtstrahlen nicht die optische Achse (in einer Seitenansicht auf die Optikanordnung gesehen). Die reflektierten Lichtstrahlen verlaufen bevorzugt in Richtung zur radialen Außenseite der Optikanordnung. Die Reflexionsschicht kann konvex oder konkav gekrümmt sein.

Die Reflexionsschicht kann in einem ringförmigen Bereich ausgebildet oder kann als ringförmige Reflexionsschicht ausgebildet sein. Der ringförmige Bereich bzw. die ringförmige Reflexionsschicht kann in sich geschlossen sein. Es ist jedoch auch möglich, dass in Umfangsrichtung und/oder in einer Richtung quer zur Umfangsrichtung eine oder mehrere Lücken vorhanden ist bzw. sind. In der Lücke bzw. in den Lücken ist bevorzugt kein Teil der Reflexionsschicht ausgebildet.

Die Aperturblende weist bevorzugt einen ersten Bereich mit der Reflexionsschicht und einen zweiten Bereich, in dem die Reflexionsschicht nicht ausgebildet ist, auf. Somit kann der erste Bereich z.B. im Wesentlichen nur Licht aus dem nahen Infrarot (und kein Licht aus dem sichtbaren Spektrum) weiterleiten und kann der zweite Bereich z.B. sowohl Licht aus dem sichtbaren Spektrum als auch Licht aus dem nahen Infrarot weiterleiten.

Die Aperturblende ist bevorzugt als transmissive Blende (bezogen auf das durch die Aperturblende weitergeleitete Licht) ausgebildet.

Durch die ringförmige Reflexionsschicht bzw. die Ausbildung der Reflexionsschicht in einem ringförmigen Bereich wird erreicht, dass der Aperturdurchmesser für Licht aus dem sichtbaren Spektrum kleiner ist als für Licht aus dem nahen Infrarot. Damit wird die gewünschte hohe Schärfentiefe für Abbildungen mit Licht aus dem sichtbaren Spektrum gewährleistet.

Des Weiteren kann der Aperturdurchmesser für Licht aus dem nahen Infrarot größer sein als für Licht aus dem sichtbaren Spektrum, so dass die gewünschte größtmögliche Transmission für Licht aus dem nahen Infrarot gewährleistet werden kann.

Die Aperturblende ist somit eine wellenlängenabhängige Aperturblende, deren Aperturdurchmesser für Licht aus dem nahen Infrarot größer sein kann als für Licht aus dem sichtbaren Spektrum.

Die Reflexionsschicht kann auf einer gekrümmten Seite eines transparenten Körpers ausgebildet sein.

Insbesondere kann die gekrümmte Seite eine sphärisch gekrümmte Seite sein. Es ist auch eine asphärische Krümmung möglich. Es ist jedoch auch jeder andere Krümmungsverlauf möglich, der verhindert, dass das reflektierte Licht aus dem sichtbaren Spektrum in sich zurückreflektiert wird.

Die beiden transparenten Körper können aus dem gleichen Material (z.B. Glas oder Kunststoff) oder aus verschiedenen Materialien (z.B. verschiedene Gläser, verschiedene Kunststoffe, Kunststoff und Glas) gebildet sein. Insbesondere können die beiden Körper als Plankonvexlinse und Plankonkavlinse ausgebildet sein.

Die Verbindung der beiden Körper kann z.B. durch Verkitten oder Ansprengen realisiert sein.

Ferner kann der näher am distalen Ende positionierte transparente Körper eine Randfläche aufweisen, die geschwärzt ist. Damit kann schon die Randfläche dieses Körpers als Strahlfalle für das reflektierte Licht aus dem sichtbaren Spektrum dienen.

Ferner kann die Optikanordnung eine Strahlfalle für das von der ringförmigen Reflexionsschicht reflektierte Licht aus dem sichtbaren Spektrum aufweisen. Diese Strahlfalle kann z.B. in einer Fassung der Aperturblende ausgebildet sein.

Die ringförmige Reflexionsschicht kann kreisringförmig ausgebildet sein. Es ist jedoch auch jede andere Ringform möglich. Insbesondere kann die Innenkontur der ringförmigen Reflexionsschicht kreisförmig, ellipsenförmig, oval, sternförmig oder polygonförmig sein.

Die Reflexionsschicht ist als dielektrische Schicht oder als dielektrisches Schichtsystem ausgebildet. Damit ist es leicht möglich, sehr exakt den Wellenlängenbereich einzustellen, der reflektiert wird, und den Wellenlängenbereich einzustellen, der transmittiert wird. Des Weiteren kann eine solche dielektrische Schicht oder ein solches dielektrisches Schichtsystem sehr dünn ausgebildet sein, was weiter vorteilhaft für die Vermeidung von unerwünschten Aberrationen für Licht aus dem sichtbaren Spektrum und/oder Licht aus dem nahen Infrarot ist.

Die Aperturblende kann insbesondere so ausgebildet sein, dass sie keine optische Brechkraft (bzw. die optische Brechkraft ist gleich Null) aufweist. So kann die Aperturblende z.B. die optische Wirkung einer planparallelen Platte aufweisen.

Ferner kann die Aperturblende in einem Abschnitt des Hauptstrahlenganges angeordnet sein, in dem ein kollimierter Strahlverlauf vorliegt.

Die Optikanordnung kann ein Objektiv am distalen Ende und ein dem Objektiv nachgeordnetes Übertragungssystem aufweisen. Die Aperturblende kann im Übertragungssystem angeordnet sein. Insbesondere kann die Aperturblende als separates Bauteil im Übertragungssystem positioniert sein. Des Weiteren ist es möglich, die Aperturblende z.B. in eine zweiteilige oder mehrteilige Stablinse zu integrieren. So kann die Reflexionsschicht z.B. auf einer gekrümmten Fläche einer Stablinse bzw. eines Elementes einer Stablinse (die bevorzugt mit einer komplementär gekrümmten Fläche eines weiteren Elementes der Stablinse verbunden ist) ausgebildet sein.

Das erfindungsgemäße Endoskop kann einen Hauptteil, einen mit dem Hauptteil verbundenen Endoskopschaft und eine erfindungsgemäße Optikanordnung (einschließlich ihrer Weiterbildungen) aufweisen, wobei die Optikanordnung bevorzugt mindestens zum Teil innerhalb des Endoskopschafts angeordnet ist.

Das erfindungsgemäße Endoskop kann als medizinisches oder technisches Endoskop ausgebildet sein. Ferner kann das erfindungsgemäße Endoskop hermetisch dicht und/oder autoklavierbar sein.

Der Endoskopschaft kann als starrer Endoskopschaft, als Endoskopschaft mit einem abwinkelbaren distalen Ende oder als flexibler Endoskopschaft ausgebildet sein.

Das erfindungsgemäße Endoskop kann an dem dem Endoskopschaft abgewandten Ende des Hauptteils einen Kameraanschluss aufweisen, an dem eine Kamera (z.B. lösbar) angebracht werden kann, mit der das mittels der Optikanordnung abgebildete Objekt aufgenommen werden kann. Die Kamera kann so ausgebildet sein, dass sie sowohl ein Bild mit Licht aus dem sichtbaren Spektrum als auch ein Bild mit Licht aus dem nahen Infrarot (gleichzeitig und/oder zeitlich nacheinander) aufnehmen kann.

Insbesondere wird ein System aus dem erfindungsgemäßen Endoskop und einer damit verbundenen Kamera bereitgestellt.

Unter Licht aus dem sichtbaren Spektrum wird hier insbesondere Licht mit einer Wellenlänge aus dem Bereich von 380 bis 750 nm und insbesondere 400 bis 700 nm verstanden. Unter Licht aus dem nahen Infrarot wird hier insbesondere Licht mit einer Wellenlänge aus dem Bereich von 780 nm bis 3 nm und insbesondere 780 nm bis 1500 nm verstanden. Auf jeden Fall überlappen sich die Wellenlängenbereiche für Licht aus dem sichtbaren Spektrum und Licht aus dem nahen Infrarot nicht.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine schematische Darstellung der erfindungsgemäßen Optikanordnung 1 in einem erfindungsgemäßen Endoskop 2;
- Fig. 2: eine schematische Darstellung der Optikanordnung 1 von Fig. 1;
- Fig. 3: eine vergrößerte Ansicht der Aperturblende 30 aus der Optikanordnung 1 gemäß Fig. 1 und 2;
- Fig. 4: eine Draufsicht der Aperturblende 30 von Fig. 3, und
- Fig. 5-10: jeweils eine Draufsicht der Aperturblende 30 gemäß weiteren Ausführungsbeispielen

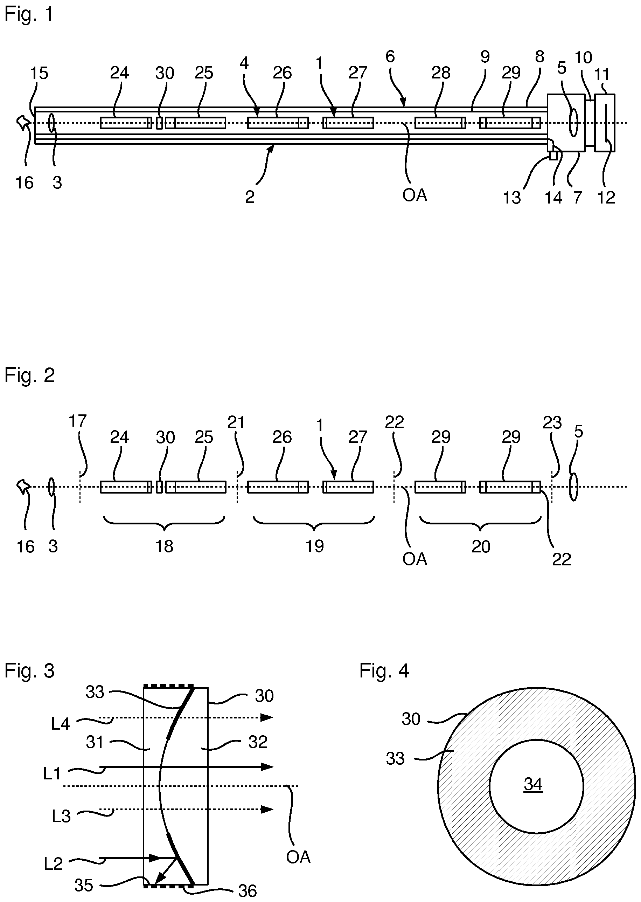

Bei der in Fig. 1 gezeigten Ausführungsform ist die erfindungsgemäße Optikanordnung 1 in einem schematisch dargestellten Endoskop 2 gezeigt. Die Optikanordnung 1 umfasst an ihrem distalen Ende ein Objektiv 3, dem eine Übertragungsoptik 4 sowie ein Okular 5 nachgeordnet sind.

Das Endoskop 2 umfasst einen Schaft 6 sowie ein damit verbundenes Hauptteil 7. Der Schaft 6 weist ein Außenrohr 8 auf, in dem ein Optikrohr 9 mit kleinerem Querschnitt sitzt. Im Optikrohr 9 ist, wie in Fig. 1 dargestellt ist, das Objektiv 3 sowie die Übertragungsoptik 4 angeordnet. Das Okular 5 ist im Hauptteil 7 positioniert.

Am vom Schaft 6 wegweisenden Ende des Hauptteils 7 kann ein Kameraanschluss 10 vorgesehen sein, an dem eine Kamera 11 lösbar befestigt ist. Die Kamera 11 kann eine (nicht gezeigte) Optik sowie einen flächigen Bildsensor 12 aufweisen. Der Bildsensor 12 kann beispielsweise ein CCD-Sensor oder ein CMOS-Sensor sein. Die Kamera 11 kann nicht nur, wie in Fig. 1 gezeigt ist, direkt mit dem Kameraanschluss 10 verbunden sein. Es ist auch möglich, dass zwischen dem Kameraanschluss 10 und der Kamera 11 ein (nicht gezeigter) Koppler zwischengeschaltet ist, der seinerseits eine Optik enthalten kann. Des Weiteren ist es möglich, dass anstatt des Kameraanschlusses 10 ein optischer Einblick (nicht gezeigt) vorgesehen ist.

Am Hauptteil 7 ist ein Beleuchtungsanschluss 13 ausgebildet, der mit Lichtleitfasern 14 (von denen in Fig. 1 stellvertretend nur eine einzige eingezeichnet ist) verbunden ist, die sich vom Beleuchtungsanschluss 13 durch einen Bereich zwischen dem Außenrohr 8 des Schaftes 6 und dem Optikrohr 9 bis zum distalen Ende 15 des Schaftes 6 erstrecken und dort das Licht zur Beleuchtung eines Objektes 16 abgeben.

Die Optikanordnung 1 ist so ausgebildet, dass das Objekt 16 auf den Bildsensor 12 abgebildet wird, wobei die Optikanordnung 1 so ausgelegt ist, dass sie sowohl für Licht aus dem sichtbaren Spektrum als auch für Licht aus dem nahen Infrarot geeignet ist. Hierunter wird z.B. verstanden, dass sowohl die Abbildung mit Licht aus dem sichtbaren Spektrum als auch die Abbildung mit Licht aus dem nahen Infrarot scharf auf den Bildsensor 12 (bzw. in die gleiche Ebene bzw. Fokusebene) erfolgt. Unter Licht aus dem sichtbaren Spektrum wird insbesondere Licht mit einer Wellenlänge aus dem Bereich von 380 bis 750 nm und insbesondere 400 bis 700 nm verstanden. Unter Licht aus dem nahen Infrarot wird hier insbesondere Licht mit einer Wellenlänge aus dem Bereich von 780 nm bis 3 µm und insbesondere 780 nm bis 1500 nm verstanden.

Das Objektiv 4, das am distalen Ende der Optikanordnung angeordnet ist, bildet das Objekt 16 in eine erste Zwischenbildebene 17 ab, die auch als distale Zwischenbildebene 17 bezeichnet werden kann (Fig. 2). Die Übertragungsoptik 4 ist als Stablinsensystem mit einer ersten, einer zweiten und einer dritten Umkehrstufe 18, 19, 20, die hintereinander angeordnet sind und jeweils ein Zwischenbild in eine nächste Zwischenbildebene abbilden, ausgebildet. So bildet die erste Umkehrstufe 18 das in der ersten Zwischenbildebene 17 liegende Zwischenbild in eine zweite Zwischenbildebene 21 ab. Das zweite Umkehrsystem 19 bildet das in der zweiten Zwischenbildebene 21 liegende Zwischenbild in eine dritte Zwischenbildebene 22 ab. Das dritte Umkehrsystem 20 bildet das Zwischenbild aus der dritten Zwischenbildebene 22 in eine vierte Zwischenbildebene 23 ab, die auch als proximale Zwischenbildebene 23 bezeichnet werden kann. Die drei Umkehrsysteme 18-20 sind somit so hintereinander angeordnet, dass ein in der distalen Zwischenbildebene 17 liegendes Zwischenbild (über die jeweils nächste Zwischenbildebene 21 und 22) in die proximale Zwischenbildebene 23 abgebildet wird. Nachdem jede Umkehrstufe 18-20 bei der Abbildung des Zwischenbildes ein umgekehrtes Zwischenbild erzeugt und eine ungerade Anzahl von Umkehrstufen 18-20 vorgesehen sind, wird das in der distalen Zwischenbildebene 17 liegende Zwischenbild des Objektes 16 als umgekehrtes Zwischenbild in die proximale Zwischenbildebene 23 abgebildet. Die Übertragungsoptik 4 kann daher auch als Umkehrsystem 4 bezeichnet werden. Das in der proximalen Zwischenbildebene 23 erzeugte Zwischenbild wird mittels des Okulars 5 auf den Bildsensor 12 abgebildet. Jede Umkehrstufe 18-20 weist hier zwei Stablinsen 24, 25 bzw. 26, 27 bzw. 28, 29 auf, die gleich oder unterschiedlich ausgebildet sein können.

Der Bereich vom Objektiv 3 bis zum Okular 5 kann als Hauptstrahlengang mit einer optischen Achse OA bezeichnet werden, durch die die Lichtstrahlen aus dem sichtbaren Spektrum und die Lichtstrahlen aus dem nahen Infrarot für die beschriebene Abbildung verlaufen.

Zwischen den Stablinsen 24 und 25 der ersten Umkehrstufe 18 ist eine Aperturblende 30 angeordnet, die in Fig. 3 vergrößert dargestellt ist. In Fig. 4 ist eine Draufsicht der Aperturblende 30 gemäß Fig. 3 gezeigt.

Die Aperturblende 30 weist eine Plankonkavlinse 31 und eine Plankonvexlinse 32 auf, deren einander zugewandten und miteinander verbundenen gekrümmten Seiten komplementäre Krümmungen aufweisen. Die Krümmungen sind jeweils sphärisch. Zwischen den beiden miteinander verbundenen gekrümmten Seiten ist eine ringförmige Reflexionsschicht 33 (die hier konvex gekrümmt ist) ausgebildet, die Licht aus dem sichtbaren Spektrum reflektiert und Licht aus dem nahen Infrarot transmittiert. Die Reflexionsschicht 33 ist in Fig. 4 schraffiert dargestellt.

Die voneinander wegweisenden Seiten der Plankonkavlinse 31 und der Plankonvexlinse 32 sind jeweils plan ausgebildet. Nachdem die beiden Linsen 31 und 32 aus dem gleichen Material gebildet sind, ist die Aperturblende 30 optisch eine planparallele Platte, die in einem Bereich des kollimierten Strahlverlaufs in der Übertragungsoptik 4 positioniert ist und daher optisch keine abbildende Wirkung aufweist. Des Weiteren ist die optische Wegstrecke für Licht aus dem sichtbaren Spektrum, das durch die Aperturblende 30 hindurchläuft, unabhängig von der Position relativ zur optischen Achse OA stets gleich. Das gleiche gilt für Licht aus dem nahen Infrarot.

Die Reflexionsschicht 33, die als dielektrische Schicht oder als dielektrisches Schichtsystem ausgebildet sein kann, ist so ausgelegt, dass sie Licht aus dem sichtbaren Spektrum (möglichst vollständig) reflektiert. Dies führt dazu, dass in einem Mittelbereich 34 der Aperturblende 30 Licht aus dem sichtbaren Spektrum transmittiert wird, wie schematisch durch den Lichtstrahl L1 angedeutet ist. Licht aus dem sichtbaren Spektrum, das außerhalb des Mittelbereiches 34 auf die Aperturblende 30 trifft, wird durch die Reflexionsschicht 33 zurückreflektiert. Da die Reflexionsschicht 33 jedoch so ausgebildet ist, dass sie gegenüber der optischen Achse OA geneigt ist (also einen Winkel von ungleich 90° aufweist), wird das Licht nicht in sich zurückreflektiert, sondern zur Seite reflektiert. Dies ist schematisch für den Lichtstrahl L2 eingezeichnet, der auf die Randfläche 35 der Plankonkavlinse 31 trifft. Bei der hier beschriebenen Ausführungsform ist die Randfläche 35 geschwärzt ausgebildet und dient als Strahlfalle 36 für die von der Reflexionsschicht 33 reflektierten Lichtstrahlen L2.

Für Licht aus dem nahen Infrarot ist die Reflexionsschicht 33 transmittierend, so dass nicht nur Licht, das auf den Mittelbereich 34 trifft, transmittiert wird, sondern auch Licht das auf die Reflexionsschicht 33 trifft. Dies ist in Fig. 3 durch die Lichtstrahlen L3 und L4 angedeutet. Damit ist der Aperturdurchmesser für Licht aus dem nahen Infrarot größer als der Aperturdurchmesser für Licht aus dem sichtbaren Spektrum. Die Aperturblende 30 ist somit eine wellenlängenabhängige Aperturblende, die einerseits die gewünschte hohe Schärfentiefe für die Abbildung mit Licht aus dem sichtbaren Spektrum (aufgrund des kleinen Aperturdurchmessers) sicherstellt. Dabei wird sicher das Erzeugen von Geisterbildern, die entstehen, wenn das Licht aus dem sichtbaren Spektrum in sich zurückreflektiert wird, verhindert. Andererseits weist die Aperurblende 30 einen größeren Durchmesser für Licht aus dem nahen Infrarot auf, was vorteilhaft ist, da in diesem Wellenlängenbereich das Signal bzw. die zur Verfügung stehende Lichtmenge häufig relativ gering ist.

Somit wird mittels der erfindungsgemäßen Aperturblende 30 in vorteilhafter Weise erreicht, dass einerseits eine gute optische Abbildungsqualität bei Licht aus dem sichtbaren Spektrum (hohe Schärfentiefe sowie die Vermeidung von Geisterbildern) sowie ein gutes Signal-RauschVerhältnis für Licht aus dem nahen Infrarot gewährleistet werden kann.

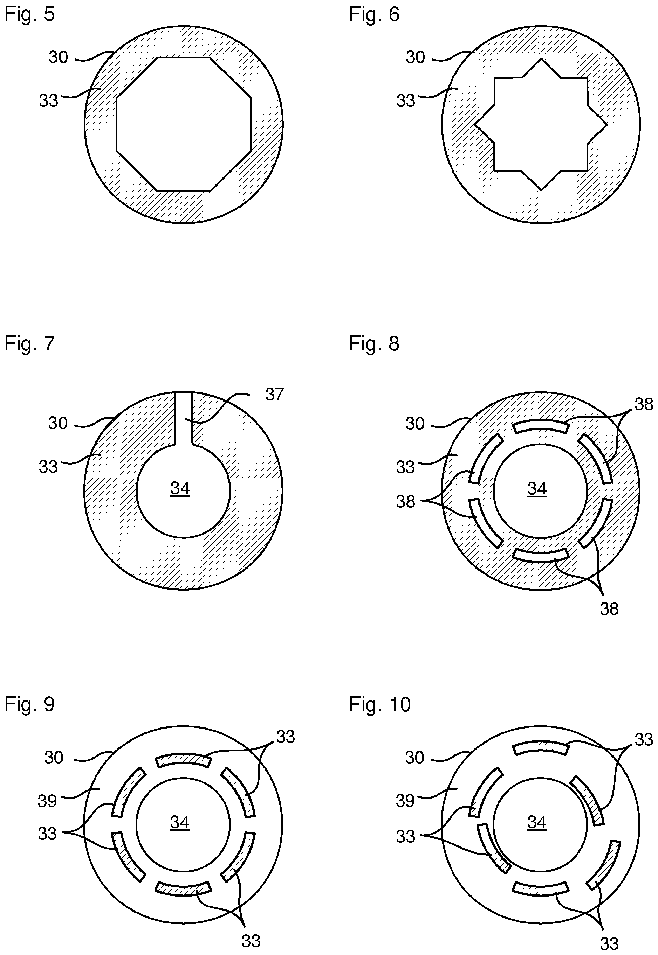

Wie in Fig. 3 und 4 gezeigt ist, ist die ringförmige Reflexionsschicht 33 als Kreisring ausgebildet. Es kann jedoch auch jede andere Ringform vorliegen. Insbesondere kann die Innenkontur der ringförmigen Reflexionsschicht 33 z.B. eine Polygonform aufweisen, wie in Fig. 5 gezeigt ist. Hier entspricht die Polygonform einem regelmäßigen Achteck. In Fig. 6 ist als Beispiel eine sternförmige Innenkontur dargestellt.

Die Reflexionsschicht 33 kann auf die konvexe Seite der Plankonvexlinse 32 oder auf die konkave Seite der Plankonkavlinse 31 aufgedampft sein. Durch diese Art der Ausbildung der Reflexionsschicht ist die Dicke der Reflexionsschicht und somit die Kante zur Begrenzung des Mittelbereiches 34 sehr dünn, so dass an dieser Kante kaum störende Aberrationen für das Licht aus dem sichtbaren Wellenlängenbereich und/oder für das Licht aus dem nahen Infrarot auftreten.

Die Schwärzung 36 der Randfläche 35 ist nur ein Beispiel für eine Strahlfalle für das reflektierte Licht aus dem sichtbaren Spektrum. Es kann auch jede andere Art der Strahlfalle genutzt werden. So kann beispielsweise der entsprechende Fassungsbereich der (nicht eingezeichneten) Fassung geschwärzt sein. Es können auch gestufte schwarz Strahlfallen ausgebildet sein.

Die gekrümmten Seiten der beiden Linsen 31 und 32 sind bevorzugt sphärisch gekrümmt. Es ist jedoch auch eine asphärische Krümmung möglich. Ferner ist es z.B. möglich, statt der Linsen 31, 32 entsprechende Axicons zu verwenden. Wesentlich ist, dass die beiden Linsen 31, 32 bzw. transparenten Körper komplementär ausgebildete zueinander gewandte Seiten aufweisen, die von der planen Form abweichen. Die Verbindung zwischen den beiden Linsen 31, 32 bzw. zwischen den beiden Körpern 31, 32 kann z.B. durch Verkitten oder Ansprengen realisiert sein.

Als Material wird bevorzugt ein Glasmaterial eingesetzt.

Bei der hier beschriebenen Ausführungsform kann der Mittelbereich 34 einen Durchmesser im Bereich von 1 bis 7 mm aufweisen. Der Außendurchmesser der Reflexionsschicht 33 kann im Bereich von 2,5 bis 12 mm liegen. Die Dicke der Aperturblende 30 entlang der optischen Achse OA kann im Bereich von 1,5 bis 2,5 mm liegen.

Die Anordnung der Aperturblende 30 in der ersten Umkehrstufe 18 ist bevorzugt. Jedoch kann die Aperturblende 30 auch an anderen Stellen in der Optikanordnung positioniert sein. Bevorzugt wird sie an einer Stelle mit kollimiertem Strahlverlauf positioniert. Ferner ist es möglich, die Aperturblende 30 direkt in eine der Stablinsen 24-29 zu integrieren. So kann z.B. die Stablinse 24 zweiteilig ausgebildet sein, wobei die einander zugewandten und miteinander verbundenen Seiten komplementäre Krümmungen aufweisen und auf einer dieser Seiten die Reflexionsschicht 33 ausgebildet ist.

Bei den bisher beschriebenen Ausführungsbeispielen ist die ringförmige Reflexionsschicht 33 stets in sich geschlossen. Es ist jedoch auch möglich, dass die Reflexionsschicht 33 z.B. eine quer zur Umfangsrichtung verlaufende Lücke 37 aufweist, in der die Reflexionsschicht 33 nicht ausgebildet ist, wie in Fig. 7 dargestellt ist. Natürlich können auch mehrere quer zur Umfangsrichtung verlaufende Lücken 37 (nicht gezeigt) vorgesehen sein.

Des Weiteren ist es möglich, dass die Reflexionsschicht 33 eine oder, wie in Fig. 8 gezeigt, mehrere in Umfangsrichtung verlaufende Lücken 38 aufweist. Bei dem in Fig. 8 gezeigten Ausführungsbeispiel sind die Lücken 38 regelmäßig angeordnet. Es kann auch eine unregelmäßige Anordnung vorliegen. Auch können sich die einzelnen Lücken 38 in ihren Abmessungen unterscheiden. Bei dem in Fig. 9 gezeigten Ausführungsbeispiel weist die Aperturblende 30 einen ringförmigen Bereich 39 auf, der den Mittelbereich 34 umgibt. Im ringförmigen Bereich 39 ist die Reflexionsschicht 33 angeordnet. Bei dem in Fig. 9 gezeigten Ausführungsbeispiel weist die Reflexionsschicht 33 mehrere Abschnitte auf, die z.B. Ringabschnitte sein können. Die Form und Abmessung der einzelnen Abschnitte der Reflexionsschicht 33 können jedoch auch unterschiedlich sein und andere Formen aufweisen. Auch ist die regelmäßige Anordnung gemäß Fig. 9 nicht notwendig. Wie in Fig. 10 gezeigt ist, ist eine unregelmäßige Anordnung der Abschnitte 33 möglich.

## Patentansprüche

1. Optikanordnung für ein Endoskop,
wobei die Optikanordnung (1) ein vor ihrem distalen Ende befindliches Objekt (16) entlang eines eine optische Achse (OA) aufweisenden Hauptstrahlengangs zum proximalen Ende der Optikanordnung (1) hin abbildet,
wobei die Optikanordnung (1) sowohl für Licht aus dem sichtbaren Spektrum als auch für Licht aus dem nahen Infrarot ausgelegt ist und im Hauptstrahlengang eine Aperturblende (30) mit einer Reflexionsschicht (33), die Licht aus dem sichtbaren Spektrum reflektiert und Licht aus dem nahen Infrarot transmittiert, aufweist,
**dadurch gekennzeichnet, dass**
die Reflexionsschicht (33) gegenüber der optischen Achse (OA) so geneigt ist,
dass Lichtstrahlen aus dem sichtbaren Spektrum, die parallel zur optischen Achse auf die Reflexionsschicht (33) treffen, so reflektiert werden, dass sie nicht mehr parallel zur optischen Achse verlaufen,
wobei die Aperturblende (30) zwei miteinander verbundene transparente Körper (31, 32) aufweist, deren einander zugewandten Seiten einen komplementären Krümmungsverlauf aufweisen und deren voneinander wegweisende Seiten plan ausgebildet sind,
wobei die Reflexionsschicht (33) zwischen den einander zugewandten Seiten der beiden Körper (31, 32) angeordnet ist,
und wobei die Reflexionsschicht (33) als dielektrische Schicht oder als dielektrisches Schichtsystem ausgebildet ist.

2. Optikanordnung nach Anspruch 1, bei der die Reflexionsschicht (33) in einem ringförmigen Bereich (39) ausgebildet ist.

3. Optikanordnung nach Anspruch 1 oder 2, bei der die Reflexionsschicht (33) ringförmig ausgebildet ist.

4. Optikanordnung nach Anspruch 3, bei der die ringförmige Reflexionsschicht (33) kreisringförmig ausgebildet ist.

5. Optikanordnung nach einem der obigen Ansprüche, bei der die Reflexionsschicht (33) auf einer gekrümmten Seite eines transparenten Körpers (31, 32) ausgebildet ist.

6. Optikanordnung nach Anspruch 5, bei der die gekrümmte Seite sphärisch gekrümmt ist.

7. Optikanordnung nach einem der obigen Ansprüche, bei der der näher am distalen Ende positionierte transparente Körper eine Randfläche (35) aufweist, die geschwärzt ist.

8. Optikanordnung nach einem der obigen Ansprüche, bei der die beiden transparenten Körper (31, 32) aus dem gleichen Material gebildet sind.

9. Optikanordnung nach einem der obigen Ansprüche, bei der die beiden transparenten Körper eine Stablinse (24, 25, 26, 27, 28, 29) bilden oder Teil einer Stablinse (24-29) sind.

10. Optikanordnung nach einem der obigen Ansprüche, bei der eine Strahlfalle (36) für das von der Reflexionsschicht (33) reflektierte Licht aus dem sichtbaren Spektrum vorgesehen ist.

11. Optikanordnung nach einem der obigen Ansprüche, bei der die Aperturlinse (30) in einem Abschnitt des Hauptstrahlenganges mit kollimiertem Strahlverlauf angeordnet ist.

12. Optikanordnung nach einem der obigen Ansprüche, bei der die Aperturblende (30) keine optische Brechkraft aufweist und/oder wobei die Optikanordnung (1) ein Objektiv (3) am distalen Ende und ein dem Objektiv (3) nachgeordnetes Übertragungssystem (4) aufweist und die Aperturblende (30) im Übertragungssystem (4) angeordnet ist.

13. Endoskop mit
einem Hauptteil (7),
einem mit dem Hauptteil (7) verbundenen Endoskopschaft (6) und
einer Optikanordnung (1) nach einem der obigen Ansprüche,
wobei die Optikanordnung (1) mindestens zum Teil innerhalb des Endoskopschafts (6) angeordnet ist.

## Claims

1. Optics arrangement for an endoscope,
wherein the optics arrangement (1) images an object (16) located in front of its distal end to the proximal end of the optics arrangement (1) along a principal beam path having an optical axis (OA),
wherein the optics arrangement (1) is designed both for light from the visible spectrum and for light from the near infrared and has an aperture stop (30) with a reflective layer (33) in the principal beam path, said reflective layer (33) reflecting light from the visible spectrum and transmits light from the near infrared,
**characterised in that**
the reflective layer (33) is inclined relative to the optical axis (OA) in such a way that light rays from the visible spectrum which strike the reflective layer (33) parallel to the optical axis are reflected in such a way that they no longer run parallel to the optical axis, wherein the aperture stop (30) comprises two interconnected transparent bodies (31, 32) whose sides facing each other have a complementary curvature profile and whose sides facing away from each other are flat,
wherein the reflective layer (33) is arranged between the mutually facing sides of the two bodies (31, 32)
and wherein the reflective layer (33) is formed as a dielectric layer or as a dielectric layer system.

2. Optics arrangement according to claim 1, wherein the reflective layer (33) is formed in an annular region (39).

3. Optics arrangement according to claim 1 or 2, in which the reflective layer (33) is formed in an annular shape.

4. Optics arrangement according to claim 3, in which the annular reflective layer (33) is formed in the shape of a circular ring.

5. Optics arrangement according to any one of the preceding claims, wherein the reflective layer (33) is formed on a curved side of a transparent body (31, 32).

6. Optics arrangement according to claim 5, wherein the curved side is spherically curved.

7. Optics arrangement according to any one of the preceding claims, wherein the transparent body positioned closer to the distal end has an edge surface (35) which is blackened.

8. Optics arrangement according to any one of the preceding claims, wherein the two transparent bodies (31, 32) are formed of the same material.

9. Optics arrangement according to any one of the preceding claims, wherein the two transparent bodies form a rod lens (24, 25, 26, 27, 28, 29) or are part of a rod lens (24-29).

10. Optics arrangement according to any one of the preceding claims, wherein a beam trap (36) is provided for the light from the visible spectrum reflected by the reflective layer (33).

11. Optics arrangement according to any one of the preceding claims, wherein the aperture lens (30) is arranged in a section of the principal beam path with a collimated beam profile.

12. Optics arrangement according to one of the preceding claims, wherein the aperture stop (30) has no optical refractive power and/or wherein the optics arrangement (1) comprises an objective (3) at the distal end and a transmission system (4) arranged downstream of the objective (3) and the aperture stop (30) is arranged in the transmission system (4).

13. Endoscope comprising
a main part (7),
an endoscope shaft (6) connected to the main part (7) and
an optics arrangement (1) according to one of the preceding claims,
wherein the optics arrangement (1) is arranged at least partly within the endoscope shaft (6).

## Revendications

1. Dispositif optique pour un endoscope,
dans lequel le dispositif optique (1) forme l'image d'un objet (16) se trouvant devant son extrémité distale le long d'un trajet principal des rayons présentant un axe optique (OA) vers l'extrémité proximale du dispositif optique (1),
le dispositif optique (1) étant conçu aussi bien pour la lumière provenant du spectre visible que pour la lumière provenant du proche infrarouge et présentant dans le trajet principal des rayons un diaphragme d'ouverture (30) avec une couche de réflexion (33) qui réfléchit la lumière provenant du spectre visible et transmet la lumière provenant du proche infrarouge,
**caractérisé en ce que**
la couche réfléchissante (33) est inclinée par rapport à l'axe optique (OA) de telle sorte que les rayons lumineux du spectre visible qui arrivent sur la couche réfléchissante (33) parallèlement à l'axe optique sont réfléchis de telle sorte qu'ils ne sont plus parallèles à l'axe optique,
le diaphragme d'ouverture (30) présentant deux corps transparents (31, 32) reliés l'un à l'autre, dont les côtés tournés l'un vers l'autre présentent un tracé de courbure complémentaire et dont les côtés s'écartant l'un de l'autre sont formés de manière plane, la couche réfléchissante (33) étant disposée entre les côtés des deux corps (31, 32) qui se font face,
et la couche de réflexion (33) étant conçue comme une couche diélectrique ou un système de couches diélectriques.

2. Dispositif optique selon la revendication 1, dans lequel la couche réfléchissante (33) est formée dans une zone annulaire (39).

3. Dispositif optique selon la revendication 1 ou 2, dans lequel la couche réfléchissante (33) est de forme annulaire.

4. Dispositif optique selon la revendication 3, dans lequel la couche réfléchissante annulaire (33) est de forme circulaire.

5. Dispositif optique selon l'une quelconque des revendications ci-dessus, dans lequel la couche réfléchissante (33) est formée sur une face incurvée d'un corps transparent (31, 32).

6. Dispositif optique selon la revendication 5, dans lequel le côté incurvé est incurvé de manière sphérique.

7. Dispositif optique selon l'une quelconque des revendications ci-dessus, dans lequel le corps transparent positionné plus près de l'extrémité distale présente une surface de bord (35) qui est noircie.

8. Dispositif optique selon l'une quelconque des revendications ci-dessus, dans lequel les deux corps transparents (31, 32) sont formés du même matériau.

9. Dispositif optique selon l'une quelconque des revendications ci-dessus, dans lequel les deux corps transparents forment une lentille en barreau (24, 25, 26, 27, 28, 29) ou font partie d'une lentille en barreau (24-29).

10. Dispositif optique selon l'une des revendications ci-dessus, dans lequel un piège à faisceau (36) est prévu pour la lumière du spectre visible réfléchie par la couche réfléchissante (33).

11. Dispositif optique selon l'une des revendications ci-dessus, dans lequel la lentille d'ouverture (30) est disposée dans une section du trajet principal du faisceau avec une trajectoire de faisceau collimatée.

12. Dispositif optique selon l'une des revendications ci-dessus, dans lequel le diaphragme d'ouverture (30) ne présente pas de puissance optique et/ou dans lequel le dispositif optique (1) présente un objectif (3) à l'extrémité distale et un système de transmission (4) disposé en aval de l'objectif (3) et le diaphragme d'ouverture (30) est disposé dans le système de transmission (4).

13. Endoscope doté
une partie principale (7),
une tige d'endoscope (6) reliée à la partie principale (7) et
un dispositif optique (1) selon l'une quelconque des revendications ci-dessus,
dans lequel le dispositif optique (1) est disposé au moins en partie à l'intérieur de la tige d'endoscope (6).
